(19)

Europäisches
Patentamt
European
Patent Office
Office européen
des brevets

(11) **EP 3 422 011 A1**

(12) **EUROPEAN PATENT APPLICATION**

(43) Date of publication:
**02.01.2019 Bulletin 2019/01**

(51) Int Cl.:
*G01N 33/86* (2006.01)   *G01N 33/49* (2006.01)
*G06F 19/12* (2011.01)   *G06F 19/00* (2018.01)

(21) Application number: **17178329.3**

(22) Date of filing: **28.06.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **BAKKER, Bart Jacob**
  **5656 AE Eindhoven (NL)**
• **VAN OOIJEN, Hendrik Jan**
  **5656 AE Eindhoven (NL)**
• **VAN DEN HAM, René**
  **5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **PARAMETER VALUE ESTIMATION IN COAGULATION SYSTEM**

(57)    The present invention relates to a method for estimating a value of a parameter of a coagulation system that is periodically supplied with an anticoagulant. The value of the parameter of the coagulation system is estimated based on concentration levels of 5 or 6 coagulation proteins derived from the coagulation system at a predetermined point in time relative to a point in time of supply of anticoagulant to the coagulation system. In one embodiment the parameter of the coagulation system is a time it takes the coagulation system to reach a state of sufficient coagulation after the periodic supply of anticoagulant to the coagulation system is discontinued and the 5 coagulation proteins are coagulation factor VII (F7), coagulation factor IX (F9), Anti-thrombin (AT), coagulation factor X (F10), and coagulation factor VIII (F8). Optionally the 6th coagulation protein is coagulation factor XI (F11).

FIG. 5

EP 3 422 011 A1

## Description

FIELD OF THE INVENTION

The present invention relates to the field of clinical decision support, and in particular, estimating a value of a parameter of a coagulation system.

BACKGROUND OF THE INVENTION

A coagulation system is a system that comprises proteins, regulators, and inhibitors that interact in order to generate a coagulation cascade or coagulation process. The coagulation cascade or coagulation process allows a liquid to be transformed to a gel that eventually forms a clot. The coagulation cascade can occur in blood and hence the blood is transformed from a liquid to a gel and the blood eventually forms a blood clot. Coagulation systems can for example be a part of the whole of a subject, e.g. a human subject, an animal subject, an artificial human subject, or an artificial animal subject, or they can also for example be simulated coagulation systems of such a subject.

Subjects that are at high risk for thrombosis are periodically supplied with anticoagulants, typically vitamin K antagonists (VKA) to lower their thrombosis risk, i.e., the risk of formation of a blood clot in a blood vessel that blocks the blood flow. Thrombosis risk can for example occur due to a malfunction in the coagulation process of the subject, e.g. a pathological condition that continuously triggers the coagulation process, or external factors, such as being bed-ridden. Various anticoagulants can be used in order to affect the coagulation process. For example, the VKA acenocoumarol decreases the availability of the reduced form of vitamin K that is needed for the synthesis of functional coagulation factors II, VII, IX, and X and anticoagulant proteins C, S, and Z in the liver of the subject.

The functional coagulation factors II, VII, IX, and X are used in the coagulation cascade in order to form a blood clot. By inhibiting the production of these functional coagulation factors by VKAs, the thrombosis tendency of subjects at risk is lowered. VKAs are given for long periods and typically subjects receive them throughout their life based on a medicinal schedule that determines anticoagulant dose, i.e. amount of VKA, and time intervals for supplying the subject with VKA.

If subjects with high thrombosis risk are scheduled for a surgery with moderate to high bleeding risk they need to discontinue their anticoagulant treatment, i.e. the periodic supply of anticoagulant, a certain period of time before the surgery is performed in order to avoid high blood loss during surgery. The anticoagulant treatment is generally stopped according to standardized guidelines, which take into account the type of anticoagulant treatment (e.g. acenocoumarol, warfarin, phenprocoumon) and its half-life in the general population of subjects. As a result of these guidelines all subjects are taken off their anticoagulant medication a fixed number of days before the date of their planned surgery.

WO 2012/172481 A1 discloses a simultaneous graphical representation, a risk of bleeding and a risk of thrombosis providing a visualized bridge therapy process. Furthermore, it discloses a computer-based prediction of the haemostatic situation of the examined blood circulation by using a combination of a biochemical model and a pharmacokinetic model for calculation or another mathematical representation of the blood circulation.

SUMMARY OF THE INVENTION

It can be seen as an object of one embodiment of the invention to provide a method, decision support device and/or system, and computer program which require less input parameters for estimating a value of a parameter of a coagulation system.

According to a first aspect of the present invention there is provided a method for estimating a value of a parameter of a coagulation system that is periodically supplied with an anticoagulant. The value of the parameter of the coagulation system is estimated based on concentration levels of a set of proteins derived from the coagulation system at a predetermined point in time relative to a point in time of supply of anticoagulant to the coagulation system.

In an embodiment of the invention, the set of proteins comprises 5 or 6 coagulations proteins. Since the value of the parameter of the coagulation system is estimated based on the concentration levels of 5 or 6 coagulation proteins derived from the coagulation system, less input values are required for the estimation. The concentration levels of the coagulation proteins can be derived from a sample of the coagulation system. The sample can be derived from the coagulation system and the concentration levels of the coagulation proteins can be determined at the predetermined point in time relative to a point in time of supply of anticoagulant to the coagulation system. In principle only one measurement is necessary and only one sample of the coagulation system needs to be taken.

In another embodiment of the invention, a second sample or further samples of the coagulation system can be taken at a later point in time or later points in time, for example for a double check. The concentration levels of the coagulation proteins of the second and further samples can be determined at respective predetermined points in time relative to a point in time of supply of anticoagulant to the coagulation system, i.e., the predetermined point in time relative to a point in time of supply of anticoagulant to the coagulation system is the same for the different samples, but the samples are taken after subsequent supplies of anticoagulant to the coagulation system in the periodic supply process. The value of the parameter can in an embodiment therefore also be estimated based on concentration levels of the coagulation proteins from more than one sample, e.g., first, second and/or further samples, i.e., the estimation can be based on various concentration levels of each of the coagulation proteins. In another embodiment, an average concentration level and its standard deviation can be calculated for each of the coagulation proteins based on the concentration levels determined from the different samples. The average concentration level can also be a weighted average taking into account the point in time the sample was taken, e.g., with higher weight for concentration levels determined from a more recently taken sample. The weighting may also be applied to the standard deviation. The weights can for example be learned from data or be predetermined, e.g., constant or time dependent weights.

In an embodiment of the invention, further samples can be derived from the coagulation system at various predetermined points in time relative to a point in time of supply of anticoagulant to the coagulation system, i.e., deriving two or more samples between two subsequent supplies of anticoagulant and determining the concentration level of the coagulation proteins, e.g., 1 hour after supply of anticoagulant to the coagulation system and 1 hour before the subsequent supply of the next dose of anticoagulant. In this case, a derived parameter describing the concentration level dynamic can for example be derived from the concentration levels determined from the samples, e.g., a concentration level gradient taken as the difference in concentration levels between two subsequent concentration level determinations divided by the time between the points of time when the concentration level determinations from the two samples derived from the coagulation system were performed.

The estimation of the value of the parameter can in one embodiment be performed at any time after the concentration levels of the 5 or 6 coagulation proteins have been determined. The concentration levels of the 5 or 6 coagulation proteins can for example be determined at a first point in time and the estimation of the value of the parameter of the coagulation system can be performed at a second point in time that can for example be several days later.

The anticoagulant periodically provided to the subject can for example be an anticoagulant of vitamin K antagonist (VKA) type, such as acenocoumarol, warfarin, or phenprocoumon. In other embodiments of the invention, the anticoagulant can for example also be heparin, low molecular weight heparin (LMWH), platelet aggregation inhibitor or any other anticoagulant.

The value of the parameter can for example be a time or duration, in particular a time it takes the coagulation system to reach a state of sufficient coagulation after the periodic supply of anticoagulant to the coagulation system is discontinued. If periodic supply of a VKA to a coagulation system is discontinued a state of sufficient coagulation is generally considered to be reached when an international normalized ratio (INR) value is below 1.5. Typical INR values in the healthy and non-VKA treated population range from 0.8 to 1.2. Hence the international normalized ratio is considered to be "normal" if the INR value is between 0.8 and 1.2, but for most surgeries an INR value of below 1.5 is regarded as providing an acceptable bleeding risk. A subject with high thrombosis risk that is periodically supplied with anticoagulants typically has a target INR value of between 2.0 and 3.0 and can have a target INR value of up to 3.5 during anticoagulant treatment, while INR values causing major bleeding risk can be as high as 8.0 to 10.0 and need immediate medical treatment, e.g., supplying the subject with vitamin K. Thus in order to have an acceptable INR value for subjects with high thrombosis risk, the periodic supply with VKA has to be discontinued a predetermined time before the surgery in order to allow for the INR value to reduce to a predetermined level, i.e., below 1.5.

In one embodiment, the method can also estimate two or more values of the parameter, one value of two or more parameters, or two or more values of two or more parameters. In other words, the method can estimate at least one value of at least one parameter. This at least one value of the at least one parameter can for example be the INR value, a time progression of the INR value or a time it takes for the coagulation system to reach an INR value below 1.5 after the periodic supply of anticoagulant, in particular VKA, to the coagulation system is discontinued. The inventors have found that concentration levels of the coagulation proteins vary between different coagulation systems, e.g. coagulation systems of subjects. Therefore, the reaction to anticoagulant treatment varies between different coagulation systems and an estimation of values of the parameters of the coagulation system which is true for one coagulation system cannot be transferred to another coagulation system. Hence it is necessary to consider the concentration levels of the 5 or 6 coagulation proteins derived from a specific coagulation system in order to determine the point in time when the INR value is below 1.5 for the specific coagulation system and hence when surgery is possible with acceptable bleeding risk.

In another embodiment, values of the parameter or parameters can for example be the time progression of the concentration levels of one of the coagulation proteins, the time progression of values of the international normalized ratio (INR) or time progression of other parameters of the coagulation system.

The coagulation system is periodically supplied with anticoagulants, i.e., predetermined amounts of anticoagulant in the form of doses are supplied to the coagulation system in predetermined time intervals. The amount of anticoagulant in each dose can for example be equal for all doses supplied to the coagulation system. Different coagulation systems can be supplied with different anticoagulant doses. It takes some time before the anticoagulant affects the coagulation system after the anticoagulant has been supplied to the coagulation system. Considering this the time intervals are chosen such that the effect of a previous anticoagulant dose is wearing off when the subsequent anticoagulant dose is supplied, i.e., the concentration levels of the coagulation proteins start increasing back to "normal", i.e., non-anticoagulated concentration levels.

The time intervals for the periodic supply of the coagulation system with the anticoagulant can have essentially the same time duration, for example 24 hours. The coagulation system can also be periodically supplied with anticoagulant in time windows, for example in a specific hour in every 24-hour time interval. The periodic supply with anticoagulant can follow a medication schedule with predetermined dose and time of supply. The predetermined point in time relative to the point in time of supply of the anticoagulant to the coagulation system can for example be a fixed time window related to the medication schedule.

The concentration levels of the 5 or 6 coagulation proteins derived from the coagulation system are expressed in mol/l. The coagulation proteins are part of the coagulation system and in particular can be included in a sample, such as a blood sample derived from the coagulation system. Various tests or measurements known to the person skilled in the art, for example coagulation factor activity assays or coagulation factor antigen tests can be performed on the blood sample in order to determine the concentration levels of the coagulation proteins. The concentration levels of the coagulation proteins mentioned in this text are the concentration levels of functional coagulation proteins. Coagulation factor activity assays measure concentration levels of functional coagulation proteins while most coagulation factor antigen tests measure the total concentration of both functional and non-functional coagulation factors in the blood sample derived from the coagulation system. In general vitamin K is needed for the synthesis of functional coagulation proteins. With lower amounts of available vitamin K in the coagulation system, e.g., through dietary intake or by intake of an anticoagulant in the form of a VKA, like warfarin or acenocoumarol, the functioning of the vitamin K affected coagulation proteins is impaired. That is, a smaller amount of functional coagulation proteins is produced while a relatively larger amount of non-functional coagulation proteins is produced. Vitamin K affected coagulation proteins are in particular coagulation factor II, coagulation factor VII, coagulation factor IX, coagulation factor X, as well as protein C, protein S, and protein Z. The functional coagulation proteins take part in the coagulation process, while the non-functional coagulation proteins essentially do not take part in the coagulation process. The concentration levels for most coagulation proteins are measured indirectly. Tests performed on the blood sample can for example comprise a measurement or a series of measurements of a coagulability of the blood sample. That is, the time it takes for forming a blood clot in the sample after the coagulation cascade is initiated by supplying the sample with a specific substance, such as tissue factor, compared to a coagulability of test samples with known concentration level of the respective coagulation protein that is being tested. The concentration levels in the blood sample and hence in the coagulation system can be derived from comparing the coagulability of the sample from the coagulation system and the test samples.

In one embodiment the method comprises the steps receiving the concentration levels of the 5 or 6 coagulation proteins derived from the coagulation system at the predetermined point in time relative to the point in time of supply of anticoagulant to the coagulation system and estimating the value of the parameter of the coagulation system based on the received concentration levels of the 5 or 6 coagulation proteins using an estimating algorithm.

The estimating algorithm can be trained by a machine learning method. The machine learning method can for example be a supervised learning method, in particular a regression, such as a generalized linear model (GLM). The machine learning method can in another embodiment be based on linear regression or artificial neural networks.

In one embodiment of the method the *glmfit*(*X, y, distr, param*1) function of MATLAB R2016a is used in order to train the estimating algorithm and to determine coefficients *b* for the estimation function of the estimating algorithm. The *X* parameters correspond to the parameters that the estimating algorithm is based on, i.e., parameters that affect the coagulation process of the coagulation system and/or that are affected by the anticoagulant. The *y* parameters correspond to a response on the *X* parameters and can for example be a time T at which the coagulation system reaches an INR value below 1.5 after the periodic supply of the anticoagulant to the coagulation system is discontinued. The *distr* parameter corresponds to an assumed error distribution used, e.g., a 'normal' distribution. The parameter *param*1 can for example be log*it'* in order to perform a logistic regression, as a logistic function is used as input. The coefficients b determined from *glmfit*(*X,T,'normal','log it'*) can be complex, i.e., having a real part and an imaginary part. The *glmval*(*b, X, 'log it'*) function of MATLAB R2016a can be used to determine a value of a parameter of the coagulation system that is modeled using the estimating algorithm. In this case the *glmval*(*b, X, 'log it'*) function calculates a value for *T* the estimated time at which the coagulation system reaches an INR value below 1.5 after the supply of the anticoagulant to the coagulation system is discontinued in dependence of the input parameters *X* using the logistic function, i.e.,

$$\hat{T}(X) = \frac{1}{1 + \exp(b_0 - \sum_i X_i \overline{b}_i)}$$

with values of input parameters $X_i$, complex value $b_0$ and the values of the complex conjugates $\overline{b}_i$ of coefficients *b*. Since the values $b_0$ and $\overline{b}_i$ of coefficients *b* are complex the logistic function yields a complex result and the real part of the logistic function, i.e., $Re(\hat{T}(X))$, is not limited to values between 0 and 1, but can have values above 1 in this case. Hence the estimating algorithm in this case can be used to estimate a time T at which the coagulation system reaches an INR value below 1.5 after the periodic supply of the anticoagulant to the coagulation system is discontinued as the value of the parameter of the coagulation system based on the received concentration levels of the 5 or 6 coagulation proteins which correspond to the input parameters $X$.

In one embodiment the estimating algorithm is generated by an estimating algorithm generation method. The estimating algorithm generation method comprises the steps:

initiating the estimating algorithm generation method with an estimated estimating algorithm and values of parameters of test coagulation systems, calculating cross validation errors of the estimated estimating algorithm based on values of one or more of the parameters of the test coagulation systems, and improving the estimated estimating algorithm by selecting parameters to be used by the estimated estimating algorithm based on the calculated cross validation errors. The steps calculating cross validation errors of the estimated estimating algorithm based on values of one or more of the parameters of the test coagulation systems, and improving the estimated estimating algorithm by selecting parameters to be used by the estimated estimating algorithm based on the calculated cross validation errors are repeated until the occurrence of a predetermined event. The estimated estimating algorithm generated in the last iteration step of the estimating algorithm generation method is the estimating algorithm.

The predetermined event can for example be that the method reaches a predetermined number of iteration steps, that the cross-validation error is below a predetermined threshold, or that a user stops the method.

The step of initiating the estimating algorithm generation method with an estimated estimating algorithm and values of parameters of test coagulation systems can comprise the steps:

providing an estimated estimating algorithm, receiving target values from the test coagulation systems, receiving input values of 5 or more parameters of each of the test coagulation systems, and storing the received input values in a test parameter pool. In this case the input values are derived from the test coagulation systems and the 5 or more parameters comprise the 5 or 6 coagulation proteins. In case of 6 coagulation proteins, the input values of 6 or more parameters of each of the test coagulation systems are received. The estimated estimating algorithm provided for the first iteration step, i.e., the initially guessed estimated estimating algorithm, can for example be based on an educated guess of the skilled person or taken from the literature.

The step of calculating cross validation errors of an estimated estimating algorithm based on values of one or more of the parameters of the test coagulation systems can comprise the steps:

moving one of the parameters of the test parameter pool to an estimation parameter pool of the estimated estimating algorithm, improving the estimated estimating algorithm based on the parameters from the estimation parameter pool using logistic regression on the estimated estimating algorithm in a leave one out cross validation, wherein estimated values are estimated by the estimated estimating algorithm and calculating a cross-validation error between the estimated values and the target values of the test coagulation systems, and moving the one of the parameters back from the estimation parameter pool to the test parameter pool. The steps moving one of the parameters of the test parameter pool to the estimation parameter pool of the estimated estimating algorithm, improving the estimated estimating algorithm based on the parameters from the estimation parameter pool using logistic regression on the estimating algorithm in a leave one out cross validation, and calculating a cross-validation error between the estimated values and the target values of the test coagulation systems, and moving the one of the parameters back from the estimation parameter pool to the test parameter pool are repeated until all parameters of the test parameter pool have been moved once to the estimation parameter pool.

The step of improving the estimated estimating algorithm by selecting parameters to be used by the estimated estimating algorithm based on the calculated cross validation errors can comprise the step moving the parameter with the lowest cross-validation error between the estimated values and the target values of the test coagulation systems permanently to the estimation parameter pool.

Hence the estimating algorithm generation method generates an estimating algorithm by selecting parameters from the test parameter pool that improve the result of the estimated estimating algorithm, i.e., parameters for which the cross validation error is the smallest. Furthermore, the estimated estimating algorithm is improved due to improvements of coefficients in the logistic regression. In the improvement process of the coefficients an overfitting is mitigated by the leave one out cross validation. In the leave one out cross validation the values of the parameters of one of the test coagulation systems are not considered in a training set used to improve the estimated estimating algorithm but instead serve as a test set. The test set can for example comprise only one value of each of the parameters of the estimation parameter pool in case that the test coagulation system has only one value for each of the parameters. The estimated estimating algorithm is improved with the training set using logistic regression and the improved estimated estimating algorithm is tested with the test set. The values of the parameters of the test set are also used to calculate the estimated value from which an error is derived by calculating the difference to the target value, i.e., the target value of the test coagulation system that forms the test set. The error is calculated for every value of the values of the parameters in this manner. The cross validation error is then calculated from the sum of squared errors between the estimated values and respective target values, i.e., from the errors derived from the estimated estimating algorithm using the respective test set in the leave one out cross validation process. In one embodiment each of the test coagulation systems has only one value for each parameter.

The predetermined event until which steps of the estimating algorithm generation method are repeated can for example also be that a predetermined number of parameters have been permanently moved to the estimation parameter pool or that all parameters have been permanently moved to the estimation parameter pool.

In one embodiment the estimating algorithm for estimating the value of the parameter of the coagulation system generated from the estimating algorithm generation method is an estimating algorithm that depends on the concentration levels of 5 or 6 coagulation proteins.

The estimating algorithm can for example be based on parameters such as the concentration level of coagulation factor II (F2), the concentration level of coagulation factor V (F5), the concentration level of coagulation factor VII (F7), the concentration level of coagulation factor VIII (F8), the concentration level of coagulation factor IX (F9), the concentration level of coagulation factor X (F10), the concentration level of coagulation factor XI (F11), the concentration level of Anti-thrombin (AT), the concentration level of Protein C (PC), the concentration level of Fibrinogen, the international normalized ratio, or the amount of anticoagulant provided with the last supply of anticoagulant to the coagulation system, e.g. in mg. All of the aforementioned parameters can be parameters of test coagulation systems from which values are supplied to the estimating algorithm generation method.

The following alternative denominations of the parameters are known to the person skilled in the art: coagulation factor I for Fibrinogen, Prothrombin for F2, Proaccelerin and labile factor for F5, Proconvertin for F7, antihemophilic factor A for F8, antihemophilic factor B and Christmas factor for F9, Stuart Prower factor for F10, and plasma thromboplastin antecedent for F11.

In one embodiment the parameter of the coagulation system is a time it takes the coagulation system to reach a state of sufficient coagulation after the periodic supply of anticoagulant to the coagulation system is discontinued. For most surgical procedures, a state of sufficient coagulation is generally considered to be reached when an INR value is below 1.5.

In one embodiment the 5 or 6 coagulation proteins are coagulation proteins that are affected by the anticoagulant and/or that affect the coagulation process of the coagulation system. Hence the 5 or 6 coagulation proteins can be coagulation proteins that are affected by the anticoagulant. The 5 or 6 coagulation proteins can also be coagulation proteins that affect the coagulation process of the coagulation system. Furthermore, the 5 or 6 coagulation proteins can be coagulation proteins that are affected by the anticoagulant and that affect the coagulation process of the coagulation system.

In one embodiment 5 of the 5 or 6 coagulation proteins are coagulation factor VII, coagulation factor IX, Anti-thrombin, coagulation factor X, and coagulation factor VIII. One of the 5 or 6 coagulation proteins can be coagulation factor XI. In another embodiment one or more of the 5 or 6 coagulation proteins can be any one of coagulation factor VII, coagulation factor IX, Anti-thrombin, coagulation factor X, and coagulation factor VIII.

The concentration levels of the 5 or 6 coagulation proteins derived from the coagulation system can be determined from a sample derived from the coagulation system at the predetermined point in time relative to the point in time of supply of anticoagulant to the coagulation system. The sample can for example be a blood sample that comprises various proteins. The blood sample can for example be derived from a human or animal subject. The blood sample can also be a simulation of a blood sample derived from a human or animal subject. The blood sample can also be an artificial blood sample of an artificial human or artificial animal subject.

The sample can be derived from the coagulation system between two subsequent supplies of anticoagulant to the system. The method can hence be performed to determine the parameter of the coagulation system while periodically supplying anticoagulants to the coagulation system. Therefore, it is not necessary to interrupt the periodic supply of anticoagulant to the coagulation system for performing the method according to an embodiment of the invention.

In another embodiment, the method can be performed after the periodic supply of anticoagulant to the coagulation system is discontinued, i.e., the concentration levels of the 5 or 6 proteins can be derived from the coagulation system at a predetermined point in time after supply of a last dose of anticoagulant.

The method can comprise the step performing an action based on the estimated value of the parameter of the coagulation system. The step of performing an action can also be based on one or more estimated values of the one or more parameters of the coagulation system. The action can for example be to determine a point in time for discontinuing periodic supply of anticoagulant, to determine a point in time for a surgery based on the point in time for discontinuing periodic supply of anticoagulant, or to provide the coagulation system with another drug, for example with another anticoagulant, a changed amount of anticoagulant, or with vitamin K.

According to a further aspect of the present invention, there is provided a decision support device and/or system. The decision support device or system comprises

a receiving unit for receiving concentration levels of a set of proteins derived from a coagulation system at a predetermined point in time relative to a point in time of supply of anticoagulant to the coagulation system wherein the set of proteins is selected using an estimation algorithm, and

a system unit for estimating a value of a parameter of the coagulation system. The receiving unit is configured to supply received concentration levels of the proteins derived from the coagulation system to the system unit. The system unit is configured to estimate the value of the parameter of the coagulation system based on concentration levels of the proteins. The system unit can also be configured to estimate one or more values of one or more parameters of the coagulation system based on concentration levels of the set of proteins.

The system unit can for example execute an estimating algorithm in order to estimate the value of the parameter of the coagulation system.

The receiving unit can for example be connected to the system unit in order to provide the received concentration levels of the coagulation proteins to the system unit. In one embodiment, the decision support device comprises a central unit which comprises the system unit and the receiving unit.

The system unit can be any device that is able to perform calculations based on received data such as concentration levels of the coagulation proteins in order to estimate the value of the parameter of the coagulation system, for example a processor, such as a central processing unit (CPU) or an integrated circuit (IC). The receiving unit can for example be connected to an external network, for example a hospital IT system that comprises data on coagulation systems, such as values of parameters of the system, e.g., concentration levels of proteins, time intervals and doses of anticoagulants, or any other parameters of the coagulation systems. The external network can further comprise values of measurements or tests such as Prothrombin time (PT), activated partial thromboplastin time (aPTT), international normalized ratio, Thrombin generation assay (TGA), coagulation factor activity assays, coagulation factor antigen tests, or any other test that determines coagulation parameters.

In one embodiment, the decision support device comprises a user interface. The user interface is configured to supply received concentration levels of the coagulation proteins derived from the coagulation system to the system unit and to receive the estimated value of the parameter of the coagulation system from the system unit. The user interface can also receive one or more estimated values of one or more parameters of the coagulation system from the system unit. The user interface is configured to receive by a user using the decision support device as input to the decision support device the concentration levels of the 5 or 6 coagulation proteins of the coagulation system and to provide to the user using the decision support device as output of the decision support device the value of the parameter of the coagulation system estimated by the system unit. The user interface can also provide the values of the parameters of the coagulation system estimated by the system unit to the user. The user interface can be connected to the receiving unit and/or the system unit, i.e., the user interface can be connected to the receiving unit, the system unit, or the receiving unit and the system unit. The user interface can for example comprise a graphical user interface, such as a touch display or any other interface that allows interaction with a user.

The decision support device can use any embodiment of the method of the present invention in order to estimate the value of the parameter of the coagulation system. The decision support device allows to present content to the user that credibly assists the user in performing a technical task by means of a continued and guided human-machine interaction process.

The decision support device can comprise one or more additional drug units that are configured for supplying a drug to the coagulation system. The additional drug units can be controlled by the user interface. Hence the user can control the additional drug units. The drug supply can also be automated in the sense that the drug supply is continued or discontinued by the additional drug units in dependence of the estimated value of the parameter of the coagulation system, e.g., the periodic supply of the anticoagulant can be automatically discontinued. In one embodiment the additional drug units automatically discontinue the periodic supply of anticoagulant if a surgery date is provided to the additional drug units and the time between the supply of the anticoagulant and the surgery is equal or below the estimated time it takes the system to reach a state of sufficient coagulation, e.g., an INR value below 1.5.

In one embodiment, the decision support device comprises a computer readable medium. The computer readable medium can comprise data, such as values of parameters of the coagulation system and in particular concentration levels of coagulation proteins. The computer readable medium can also comprise algorithms, such as the estimating algorithm and an algorithm of the estimating algorithm generation method.

In a further aspect of the present invention a computer program for estimating a value of a parameter of a coagulation system is presented. The computer program comprises program code means for causing a processor to carry out the method as defined in claim 1, when the computer program is run on the processor.

Other embodiments of the computer program can comprise program code means for causing the processor to carry out the method as defined in any of the dependent claims. The computer program can also be configured to be executed on the decision support device.

In a further aspect of the present invention a computer readable medium is presented. The computer readable medium comprises a computer program according to claim 14.

Other embodiments of the computer readable medium can comprise embodiments of the computer programs that comprise computer program code means for causing a processor to carry out the method as defined in any of the dependent claims. The computer readable medium can be configured to be used in a system connectable to the decision support device, e.g., a personal computer or the external network, or it can be part of the decision support device or used in the decision support device, e.g., if the receiving unit is configured for receiving data from the computer readable medium.

According to yet a further aspect of the invention there is provided a method for estimating a value of a parameter of a coagulation system that is periodically supplied with an anticoagulant, wherein the value of the parameter of the coagulation system is estimated based on concentration levels of 5 or 6 coagulation proteins derived from the coagulation system at a predetermined point in time relative to a point in time of supply of anticoagulant to the coagulation system.

According to yet a further aspect of the invention there is provided a decision support system comprising: a receiving unit for receiving concentration levels of coagulation proteins derived from a coagulation system at a predetermined point in time relative to a point in time of supply of anticoagulant to the coagulation system, and a system unit for estimating a value of a parameter of the coagulation system, wherein the receiving unit is configured to supply received concentration levels of the coagulation proteins derived from the coagulation system to the system unit, and wherein the system unit is configured to estimate the value of the parameter of the coagulation system based on concentration levels of 5 or 6 coagulation proteins.

These and other aspects of the invention will be apparent from and elucidated with reference to the embodiments described hereinafter.

BRIEF DESCRIPTION OF THE DRAWINGS

Embodiments of the invention are described by way of example with reference to the following drawings:

Fig. 1 shows a flow diagram of a first embodiment of the method,
Fig. 2 shows a flow diagram of a second embodiment of the method,
Fig. 3 shows a flow diagram of an embodiment of the estimating algorithm generation method for generating the estimating algorithm,
Fig. 4 shows schematically an embodiment of the decision support device and system,
Fig. 5 shows a graph of sum-squared estimation error calculated with the estimating algorithm in dependence of parameters used by the estimating algorithm,
Fig. 6 shows a graph of a study of 12 individuals with estimated and measured times until INR reaches a value below 1.5 after discontinuation of periodic supply with anticoagulant.

DETAILED DESCRIPTION OF EMBODIMENTS

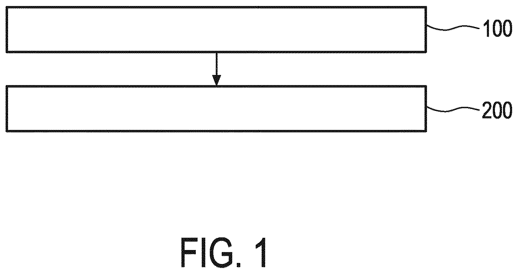

Fig. 1 shows a first embodiment of a flow diagram of the method for estimating a time it takes a coagulation system that is periodically supplied with an anticoagulant, in particular a VKA, to reach a state of sufficient coagulation, in this case an INR value below 1.5, after the periodic supply of VKA to the coagulation system is discontinued. This time can for example be used to plan a surgery or determine the point in time when the periodic supply of VKA should be discontinued in order to have an acceptable bleeding risk at a surgery planned for a specific date.

The time it takes for the INR to reach a value below 1.5 is estimated based on concentration levels of 5 or 6 coagulation proteins, i.e., concentration levels of functional coagulation proteins, derived from the coagulation system at a predetermined point in time relative to a point in time of supply of VKA to the coagulation system. The 5 or 6 coagulation proteins are coagulation proteins that are affected by the anticoagulant, in this embodiment a VKA, and/or that affect the coagulation process of the coagulation system. In particular, the coagulation proteins are F7, F9, AT, F10, and F8. The 6th coagulation protein F11 is optional.

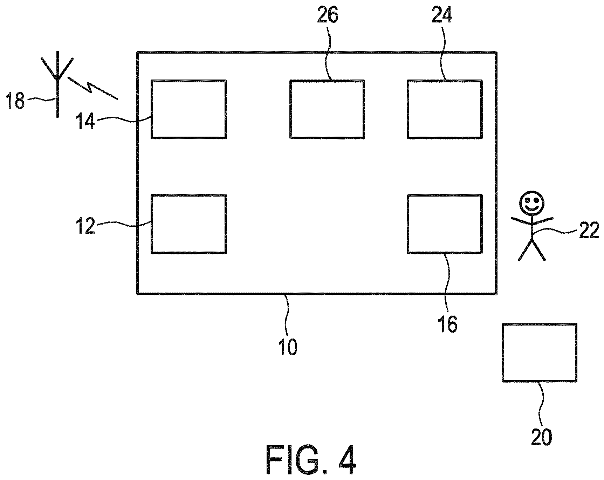

The method can for example be performed on a decision support device 10 as presented in Fig. 4 or any other device that can execute an algorithm, e.g., a personal computer. In step 100 of Fig. 1 the concentration levels of the 5 or 6 coagulation proteins derived from the coagulation system at the predetermined point in time relative to the point in time of supply of VKA to the coagulation system are received. In step 200 the time it takes for the INR to reach a value below 1.5 after the periodic supply of VKA to the coagulation system is discontinued is estimated based on the received concentration levels of the 5 or 6 coagulation proteins using an estimating algorithm. The estimating algorithm can for example be executed on a system unit 14 of the decision support device 10.

The estimating algorithm is trained by a machine learning method. In this embodiment the machine learning method is a supervised learning method, in particular a regression in the form of a generalized linear model.

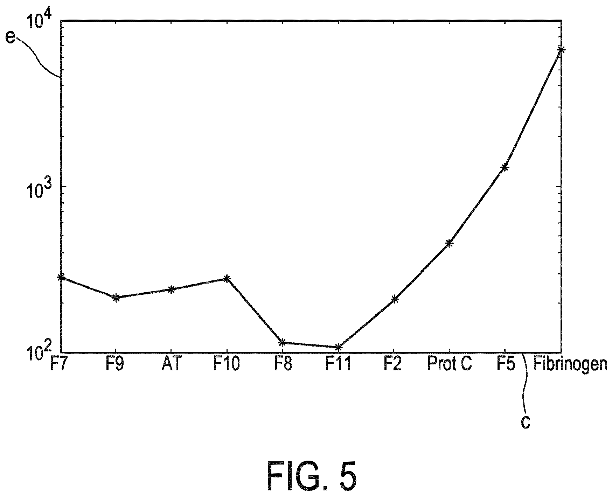

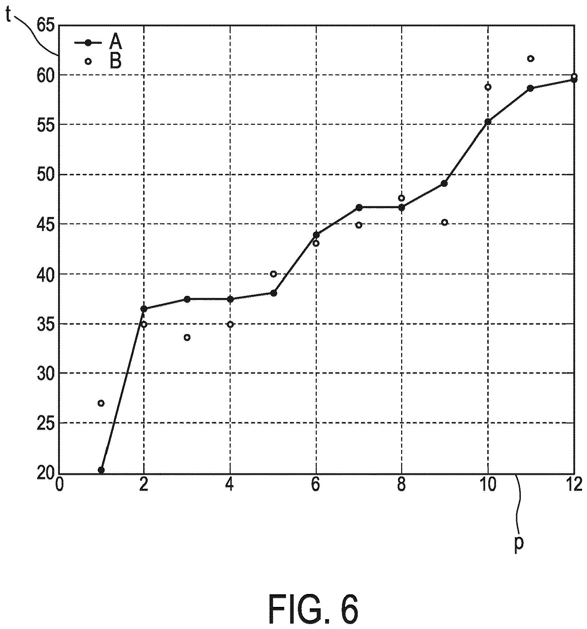

In order to derive input values for the supervised learning method a study was performed (see Fig. 5 and 6). The daily progression of the INR value of 12 individual subjects was recorded for 8 days after discontinuation of periodic supply of VKA. From this time progression of the INR values a time at which the INR value crossed 1.5 for each of the subjects was determined by linear interpolation between the derived daily values to the nearest minute. A target value $T$ was determined as the time between the interpolated time and the point in time of the last supply with a VKA. In the study acenocoumarol is used as VKA. In other embodiments any other vitamin K antagonist type of anticoagulant can be used. Yet in other embodiments, in particular for other affected coagulation proteins, non vitamin K antagonist type anticoagulants can be used. The input concentration levels of F2, F5, F7, F8, F9, F10, F11, AT, PC, and Fibrinogen, as well as the INR value were determined from a blood sample drawn within the first 12 hours after the last dose of acenocoumarol was supplied to the subject. The amount of acenocoumarol of the last dose supplied to the subject was recorded. The aforementioned parameters were used as inputs to the GLM model.

The method for training the estimating algorithm is based on the *glmfit*($X$, y, *distr, param*1) function of MATLAB R2016a, which is used in order to train the estimating algorithm and to determine coefficients $b$ for the estimation function of the estimating algorithm.

The $X$ parameters correspond to parameters that the estimating algorithm is based on, i.e., the input concentration levels of F2, F5, F7, F8, F9, F10, F11, AT, PC, and Fibrinogen, as well as the INR value and the amount of acenocoumarol of the last dose.

The $y$ parameters correspond to a response on the $X$ parameters and are in this case the times T at which the coagulation systems reach an INR value below 1.5 after the periodic supply of the acenocoumarol to the coagulation systems is discontinued.

The *distr* parameter corresponds to an assumed error distribution used. In this embodiment a 'normal' distribution is used.

The parameter *param*1 in this embodiment is 'log*it*' in order to perform a logistic regression, as a logistic function is used as input.

The coefficients $b$ determined from *glmfit*($X$, $T$, 'normal', 'log*it*') can be complex, i.e., having a real part and an imaginary part. The *glmval*($b$, $X$, 'log*it*') function of MATLAB R2016a is used to calculate a value for $\hat{T}$ the estimated time at which the coagulation system reaches an INR value below 1.5 after the periodic supply of the acenocoumarol to the coagulation system is discontinued in dependence of the input parameters $X$ using the logistic function, i.e.,

$$\hat{T}(X) = \frac{1}{1 + \exp(b_0 - \sum_i X_i \bar{b}_i)}$$

with values $X_i$ of the input parameters $X$, complex value $b_0$ and the values of the complex conjugates $\bar{b}_i$ of coefficients $b$. Since the values $b_0$ and $\bar{b}_i$ of the coefficients $b$ are complex also the logistic function yields a complex result and the real part of the logistic function, i.e., $\text{Re}(\hat{T}(X))$, is not limited to values between 0 and 1, but can have values above 1 in this case.

Hence the estimating algorithm in this case can be used to estimate a time $\hat{T}$ at which the coagulation system reaches an INR value below 1.5 after the periodic supply of the acenocoumarol to the coagulation system is discontinued. The GLM method finds that using the concentration levels of the aforementioned 5 or 6 coagulation proteins as input to the estimating algorithm yields the best estimated values, i.e., the values with lowest difference to the target values determined from the study.

The method can also be used to estimate other values of other parameters of the coagulation system, such as a time progression of the concentration levels of one or more of the coagulation proteins, the time progression of values of the INR or time progression of other parameters of the coagulation system.

For performing the method, the estimating algorithm can be used with the concentration levels of the 5 or 6 coagulation proteins as input. The concentration levels of the 5 or 6 coagulation proteins can therefore be determined from a sample, in particular a blood sample, derived from the coagulation system at the predetermined point in time relative to the point in time of supply of VKA to the coagulation system.

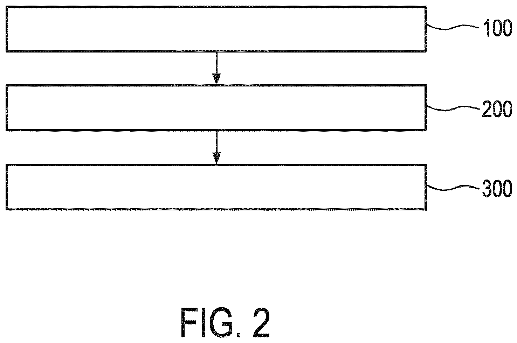

Fig. 2 shows schematically and exemplarily a second embodiment of the method for estimating a time it takes a coagulation system that is periodically supplied with an anticoagulant to reach an INR value below 1.5 after the periodic supply of anticoagulant, in particular a VKA, to the coagulation system is discontinued. The second embodiment is similar to the first embodiment of the method, but with the difference that the second embodiment comprises the additional step 300 of performing an action based on the estimated time it takes the coagulation system to reach an INR value below 1.5 after the periodic supply of VKA to the coagulation system is discontinued.

The action in this embodiment is to determine a point in time at which bleeding risk is acceptable for a surgery. Other actions can for example be to determine a point in time for discontinuing periodic supply of VKA, or to provide the coagulation system with another drug, for example with another anticoagulant, a changed amount of anticoagulant, or with vitamin K.

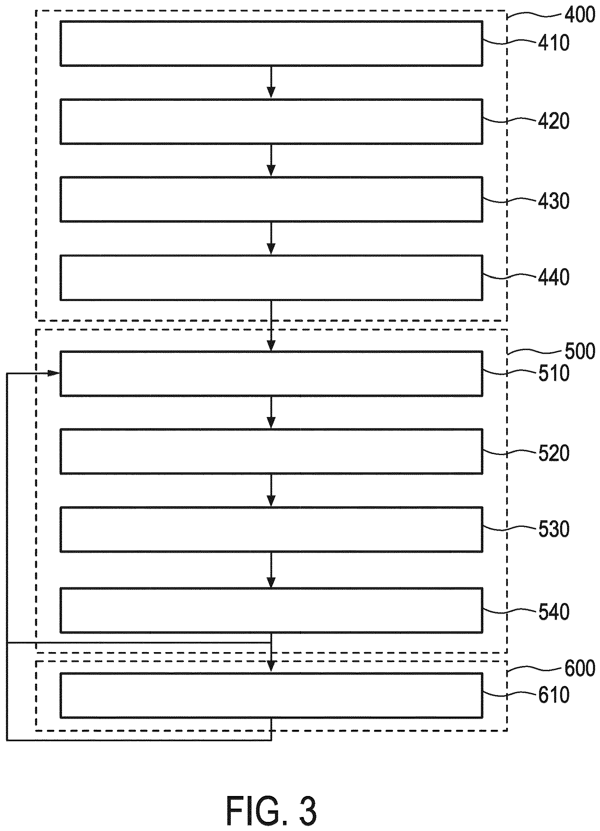

Fig. 3 shows an embodiment of an estimating algorithm generation method. An estimating algorithm is iteratively generated by the estimating algorithm generation method. Therefore, an improved estimated estimating algorithm is generated in every iteration step which is used to estimate the value of the parameter. The difference between estimated values and target values is compared in order to determine the quality of the estimated estimating algorithm. The estimated estimating algorithm generated in the last iteration step is used as estimating algorithm. The embodiment of the algorithm generation method is initiated with an estimated estimating algorithm and values of parameters of test coagulation systems in step 400. In step 500 cross validation errors of an estimated estimating algorithm based on values of one or more of the parameters of the test coagulation systems are calculated. In step 600 the estimated estimating algorithm is improved by selecting parameters to be used by the estimated estimating algorithm based on the calculated cross validation errors.

The steps 500 and 600 are repeated until a user stops the method. Steps 500 and 600 can also be repeated until the occurrence of any other predetermined event, e.g., that the method reaches a predetermined number of iteration steps, that the cross-validation error is below a predetermined threshold or that a predetermined number of parameters has been selected, e.g. 5, 6, or 10.

In this embodiment values of parameters of 12 test coagulation systems are provided. In other embodiments another number of test coagulation systems can be considered.

In this embodiment step 400 comprises sub steps 410 to 440. In step 410 an estimated estimating algorithm is provided. In step 420 target time values from the test coagulation systems are received. In step 430 input values of 12 parameters of each of the test coagulation systems are received. In step 440 the received input values are stored in a test parameter pool. The steps 410 to 430 can also be interchanged, as it does not matter whether target time values or input values are received first or whether an estimated estimating algorithm is provided before the values are received. The target time values and input values can also be received at the same point of time, e.g. in a data package.

In this embodiment the estimated estimating algorithm provided for initializing the estimating algorithm generation method is an algorithm that estimates a value for T the estimated time at which the coagulation system reaches an INR value below 1.5 after the supply of VKA to the coagulation system is discontinued in dependence of some of the 12 input parameters $X$ with value $X_i$ of the $i$ -th input parameter using the logistic function, i.e.,

$$\hat{T}(X) = \frac{1}{1 + \exp(b_0 - \sum_i X_i \bar{b}_i)}$$

with complex value $b_0$ and values of the complex conjugates $\bar{b}_i$ of coefficients $b$. It is iteratively determined which of the input parameters $X$ are to be considered by the estimated estimating algorithm in order to improve the estimated estimating algorithm. Furthermore the values $b_0$ and $\bar{b}_i$ of the coefficients $b$ are iteratively improved by the estimating algorithm generation method in order to improve the estimated estimating algorithm.

In this embodiment the input values are derived from the 12 test coagulation systems. In other embodiments the input values can be derived from a different number of test coagulation systems. The 12 parameters $X_i$ are the received concentration levels of F2, F5, F7, F8, F9, F10, F11, AT, PC, and Fibrinogen, as well as the INR value and the amount of anticoagulant of the last dose. The received concentration levels are the concentration levels of the functional coagulation proteins. In another embodiment a derived parameter, such as the concentration level gradient can be one of the parameters. Furthermore, more than 12 parameters can be received and considered in the estimating algorithm generation method.

In this embodiment step 500 comprises sub steps 510 to 540. In step 510 one of the parameters of the test parameter pool is moved to an estimation parameter pool of the estimated estimating algorithm. In step 520 the estimated estimating algorithm is improved based on the parameters from the estimation parameter pool using logistic regression on the estimated estimating algorithm in a leave one out cross validation. Furthermore, estimated values are estimated by the estimated estimating algorithm. In step 530 a cross-validation error between the estimated values and the target values of the test coagulation systems are calculated. In step 540 the parameter moved to the estimation parameter pool in step 510 is moved back from the estimation parameter pool to the test parameter pool. The steps 510 to 540 are repeated until all parameters of the test parameter pool have been moved once to the estimation parameter pool.

In this embodiment step 600 comprises sub step 610. In step 610 the parameter with the lowest cross-validation error between the estimated values and the target values of the test coagulation systems is permanently moved to the estimation parameter pool.

From this estimating algorithm generation method an estimating algorithm is generated that depends on 5 or 6 coagulation proteins, namely in this embodiment F7, F9, AT, F10, and F8 and optionally F11.

Fig. 4 shows an embodiment of a decision support device and/or system 10. The decision support device comprises a receiving unit 12 and a system unit 14.

The receiving unit 12 receives concentration levels of coagulation proteins derived from a coagulation system 20 at a predetermined point in time relative to a point in time of supply of anticoagulant to the coagulation system 20. The receiving unit 12 is connected to the system unit 14 in this embodiment. In other embodiments receiving unit 12 and system unit 14 can also be combined in a central unit (not shown). The receiving unit 12 supplies the received concentration levels of the coagulation proteins derived from the coagulation system 20 to the system unit 14.

The system unit 14 estimates one or more values of one or more parameters of the coagulation system 20 based on concentration levels of 5 or 6 coagulation proteins using the estimating algorithm. In particular, the system unit 14 can perform each of the embodiments of the method as described for Fig. 1 and 2. The system unit 14 can further generate the estimating algorithm performing the estimating algorithm generation method described for Fig. 3.

In this embodiment the decision support device 10 further comprises a user interface 16. The user interface 16 is a graphical user interface in this embodiment that allows a user 22 to interact with the decision support device 10. Therefore, the user interface 16 has a display and buttons. In other embodiments the user interface 16 can also be any other kind of user interface 16 that allows an interaction with a user 22, for example an audio interface that allows to enter information through voice or a touch display that allows to enter information by physical interaction with the touch display.

The user interface 16 is connected to the receiving unit 12 and the system unit 14. The user interface 16 receives the concentration levels of the 5 or 6 coagulation proteins as input to the decision support device 10 from the user 22. The user interface 16 provides the received concentration levels of the coagulation proteins to the system unit 14. The system unit 14 estimates a time it takes for the coagulation system 20 to reach an INR value below 1.5 after periodic supply of anticoagulant is discontinued. The system unit 14 supplies the estimated time value to the user interface 16. The user interface 16 provides to the user 22 the time value estimated from the system unit 14 as output. The time value estimated by the system unit 14 is therefore displayed to the user 22 which can perform an action based on the estimated time value.

In another embodiment the decision support device 10 there is no user interface 16 present (not shown). Hence a user interface 16 is only optional. Instead the user interface 16 can be replaced by an audio unit or a visual display unit such as a display, a screen, or a monitor. The interaction with the decision support device 10 can in this case for example be enabled by an external device connected to the decision support device 10 (not shown).

In this embodiment the receiving unit 14 is wirelessly connected to an external network 18. The external network 18 in this embodiment is a hospital IT system. The hospital IT system comprises data on coagulation systems, in particular coagulation systems of subjects. Hence various values of parameters of the coagulation system 20 can be supplied to the decision support device 10 via the external network 18.

In one embodiment the decision support device 10 includes a computer readable medium 24 that comprises a computer program that comprises program code means that enable the system unit 14 to perform the embodiments of the methods described for Fig. 1 and 2, as well as the embodiment of the estimating algorithm generation method described for Fig. 3. The computer readable medium 24 is optional. Program code means can for example also be received via the external network 18 or they can also be stored on internal memory of the decision support device 10 (not shown).

In one embodiment the decision support device 10 includes an additional drug unit 26. The additional drug unit 26 can be controlled via the user interface 16. The additional drug unit 26 allows supplying a drug to the coagulation system 20. In particular, the additional drug unit 26 can be programmed to automatically supply the coagulation system 20 with anticoagulant according to a medical schedule comprising anticoagulant dose and time intervals of dosage. Hence the additional drug unit 26 can periodically supply the coagulation system 20 with the anticoagulant. The additional drug unit 26 can also be controlled by the system unit 14 which can perform an action based on the predicted values to supply a drug or discontinue supply of a drug, e.g., an anticoagulant or vitamin K. The additional drug unit 26 is optional. Drug supply can for example also be manually controlled or performed. In other embodiments more than one additional drug unit 26 may be part of the decision support device 10 or connected to the decision support device 10. An additional drug unit connected to the decision support device 10 may be controlled by the decision support device 10 (not shown).

In this embodiment the coagulation system 20 is a coagulation system of a subject. The coagulation system 20 can also be a simulation of a coagulation system of a subject or an artificial coagulation system, such as a system mimicking the functions of a coagulation system in form of a coagulation system of a subject, or an artificial subject corresponding to an artificial coagulation system.

Fig. 5 shows a graph of sum-squared estimation error e calculated with the estimating algorithm as a function of the number of selected parameters c included in the estimation parameter pool of the estimating algorithm used for estimating the value of the parameter of the coagulation system. The vertical axis indicates the sum-squared estimation error e between estimated values and target values of the test coagulation systems and the horizontal axis indicates from left to right the cumulative parameters permanently moved to the estimation parameter pool, i.e., which parameter has been moved to the estimation parameter pool in which iteration step of the estimating algorithm generation method described for Fig. 3. The data is obtained from the study with 12 individual subjects as described for Fig. 1.

The sum-squared estimation error has the lowest values when 5 or 6 parameters are used by the estimating algorithm in order to estimate a time value for the time it takes for the coagulation system to reach an INR value below 1.5 after the periodic supply of acenocoumarol to the coagulation system is discontinued. In particular, the 5 coagulation proteins coagulation factor VII F7, coagulation factor IX F9, Anti-thrombin AT, coagulation factor X F10, and coagulation factor VIII F8 yield good results. Optionally the 6th coagulation protein coagulation factor XI F11 can be used as a parameter to further decrease the sum-squared estimation error. If more parameters are used the sum-squared estimation error e increases. This behavior can be associated with an overfitting of the model. Therefore, choosing the concentration levels of the aforementioned 5 or 6 coagulation proteins as input for the estimating algorithm yields the best results.

Fig. 6 shows a graph of the study of 12 individuals with estimated times B and measured times A until INR reaches a value below 1.5 after discontinuation of anticoagulation treatment, i.e., periodic supply of anticoagulant, in this case acenocoumarol, a VKA. The vertical axis indicates the time t in hours and the horizontal axis lists the subject number up from 1 to 12. The estimated times B are calculated using the embodiment of the method as presented in Fig. 1 based on the concentration levels of the 5 coagulation proteins F7, F9, AT, F10, and F8. For the study the concentration levels of the coagulation proteins are derived from blood samples taken from the individual subjects at a predetermined point in time relative to the point in time of the supply of the last dose of anticoagulant to the coagulation system. In particular, the blood samples were taken within 12 hours of the last dose of acenocoumarol.

The graph shows decent agreement of the estimated values and the measured time values. According to guideline recommendation, e.g., as provided by James D. Douketis et al. in "Perioperative Management of Antithrombotic Therapy: Antithrombotic Therapy and Prevention of Thrombosis, 9th ed: American College of Chest Physicians Evidence-Based Clinical Practice Guidelines" periodic supply of vitamin K antagonists to subjects shall be discontinued 5 days in advance of a surgery, i.e., 120 hours before the surgery. The guideline recommendation therefore has a worse agreement compared to the measured time values than the values estimated by the estimating algorithm. Hence using the method according to an embodiment of the invention allows reducing the thrombosis risk of a subject, as the coagulation system is untreated with anticoagulant for a shorter period of time.

While the invention has been illustrated and described in detail in the drawings and foregoing description, such illustration and description are to be considered illustrative or exemplary and not restrictive; the invention is not limited to the disclosed embodiments. For example, it is possible to operate the invention in an embodiment wherein more than one drug interaction, e.g., with two different anticoagulants, is studied in order to predict one or more values of one or more parameters of the system. In particular, the method can be operated in an embodiment wherein a first anticoagulant affects the INR value and a second anticoagulant does not affect the INR value, such as heparin, low molecular weight heparin, platelet aggregation inhibitors, et cetera. Furthermore, for example other factors can be considered for estimating one or more values of one or more parameters of the coagulation system, such as the time it takes to reach an INR of 1.5. Other factors can for example include the production of VKA in a coagulation system and in particular in a liver of a coagulation system by metabolization, the concentration levels of liver enzymes aspartate transaminase (AST) and alanine transaminase (ALT), which indicate the functioning of the liver and hence, may provide an indication about the speed of elimination of VKA, as well as an age of a coagulation system which influences the elimination of VKA. The method can in principle be used to estimate a time at which a surgery can be safely performed after periodic supply of anticoagulant is discontinued.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit, processor, or device may fulfill the functions of several items recited in the claims. The mere fact that certain measures are recited in mutually different dependent claims does not indicate that a combination of these measures cannot be used to advantage.

Operations like receiving concentration levels, estimating the value of the parameter of the coagulation system, initiating the estimating algorithm generation method, calculating cross validation errors, improving the estimated estimating algorithm, performing an action based on the estimated value of the parameter of the system, et cetera performed by one or several units or devices can be performed by any other number of units or devices. These operations and/or the control of the decision support device can be implemented as program code means of a computer program and/or as dedicated hardware.

A computer program may be stored/distributed on a suitable medium, such as an optical storage medium, or a solid-state medium, supplied together with or as part of other hardware, but may also be distributed in other forms, such as via the Internet or other wired or wireless telecommunication systems.

Any reference signs in the claims should not be construed as limiting the scope.

An embodiment of the invention relates to a method for estimating a value of a parameter of a coagulation system that is periodically supplied with an anticoagulant. The value of the parameter of the coagulation system is estimated based on concentration levels of 5 or 6 coagulation proteins derived from the coagulation system at a predetermined point in time relative to a point in time of supply of anticoagulant to the coagulation system. In one embodiment the parameter of the coagulation system is a time it takes the coagulation system to reach a state of sufficient coagulation after the periodic supply of anticoagulant to the coagulation system is discontinued and the 5 coagulation proteins are coagulation factor VII, coagulation factor IX, Anti-thrombin, coagulation factor X, and coagulation factor VIII. Optionally the 6th coagulation protein is coagulation factor XI.

**Claims**

1.  A method for estimating a value of a parameter of a coagulation system that is periodically supplied with an anticoagulant, wherein the value of the parameter of the coagulation system is estimated based on concentration levels of a set of proteins derived from the coagulation system at a predetermined point in time relative to a point in time of supply of anticoagulant to the coagulation system, and wherein the set of proteins is selected using an estimation algorithm.

2.  The method according to claim 1, wherein the set of proteins comprises 5 or 6 coagulation proteins.

3.  The method according to claim 2, wherein the method comprises the steps
    receiving the concentration levels of the 5 or 6 coagulation proteins derived from the coagulation system at the predetermined point in time relative to the point in time of supply of anticoagulant to the coagulation system,
    estimating the value of the parameter of the coagulation system based on the received concentration levels of the 5 or 6 coagulation proteins using the estimating algorithm.

4.  The method according to any preceding claim, wherein the estimating algorithm is trained by a machine learning method.

5.  The method according to any of claims 1 to 4, wherein the estimation algorithm is generated by an iterative method.

6.  The method according to any of claims 1 to 4, wherein the estimating algorithm is generated by an estimating algorithm generation method comprising the steps:

    initiating the estimating algorithm generation method with an estimated estimating algorithm and values of parameters of a test coagulation system,
    calculating cross validation errors of the estimated estimating algorithm based on values of one or more of the parameters of the test coagulation system, and

improving the estimated estimating algorithm by selecting parameters to be used by the estimated estimating algorithm based on the calculated cross validation errors,
wherein the steps of calculating cross validation errors and improving the estimated estimating algorithm are repeated until the occurrence of a predetermined event, and wherein the estimated estimating algorithm generated in the last iteration step of the estimating algorithm generation method is the estimating algorithm.

7.  The method according to claim 6, wherein the step of initiating the estimating algorithm generation method with an estimated estimating algorithm and values of parameters of test coagulation systems comprises the steps:

    providing an estimated estimating algorithm,
    receiving target values from the test coagulation systems,
    receiving input values of 5 or more parameters of each of the test coagulation systems, and storing the received input values in a test parameter pool, wherein the input values are derived from the test coagulation systems and wherein the 5 or more parameters comprise the 5 or 6 coagulation proteins, and
    wherein the step calculating cross validation errors of the estimated estimating algorithm based on values of one or more of the parameters of the test coagulation systems comprises the steps: moving one of the parameters of the test parameter pool to an estimation parameter pool of the estimated estimating algorithm,
    improving the estimated estimating algorithm based on the parameters from the estimation parameter pool using logistic regression on the estimated estimating algorithm in a leave one out cross validation, wherein estimated values are estimated by the estimated estimating algorithm and
    calculating a cross-validation error between the estimated values and the target values of the test coagulation systems, and
    moving the one of the parameters back from the estimation parameter pool to the test parameter pool,
    wherein the step of moving one of the parameters of the test parameter pool to the estimation parameter pool of the estimated estimating algorithm, improving the estimated estimating algorithm based on the parameters from the estimation parameter pool using logistic regression on the estimating algorithm in a leave one out cross validation, calculating a cross-validation error between the estimated values and the target values of the test coagulation systems, and

moving the one of the parameters back from the estimation parameter pool to the test parameter pool are repeated until all parameters of the test parameter pool have been moved once to the estimation parameter pool, and

wherein the step of improving the estimated estimating algorithm by selecting parameters to be used by the estimated estimating algorithm based on the calculated cross validation errors comprises the step:

moving the parameter with the lowest cross-validation error between the estimated values and the target values of the test coagulation systems permanently to the estimation parameter pool.

8. The method according to claim 1, wherein the parameter of the coagulation system is a time it takes the coagulation system to reach a state of coagulation after the periodic supply of anticoagulant to the coagulation system is discontinued.

9. The method according to claim 2, wherein the 5 or 6 coagulation proteins are coagulation proteins that are affected by the anticoagulant, that affect the coagulation process of the coagulation system, or that are affected by the anticoagulant and affect the coagulation process of the coagulation system.

10. The method according to claim 2, wherein 5 of the 5 or 6 coagulation proteins are coagulation factor VII (F7), coagulation factor IX (F9), Anti-thrombin (AT), coagulation factor X (F10), and coagulation factor VIII (F8).

11. The method according to claim 9 or 10, wherein one of the 5 or 6 coagulation proteins is coagulation factor XI (F11).

12. The method according to claim 2, wherein the concentration levels of the 5 or 6 coagulation proteins derived from the coagulation system are determined from a sample derived from the coagulation system at the predetermined point in time relative to the point in time of supply of anticoagulant to the coagulation system.

13. A decision support device (10) comprising:

a receiving unit (12) for receiving concentration levels of a set of proteins derived from a coagulation system (20) at a predetermined point in time relative to a point in time of supply of anticoagulant to the coagulation system wherein the set of proteins is selected using an estimation algorithm, and

a system unit (14) for estimating a value of a parameter of the coagulation system (20), wherein the receiving unit (12) is configured to supply received concentration levels of the proteins derived from the coagulation system (20) to the system unit (14), and

wherein the system unit (14) is configured to estimate the value of the parameter of the coagulation system (20) based on concentration levels of the set of proteins.

14. The device (10) according to claim 13 comprising a user interface (16), wherein the user interface (16) is configured to supply received concentration levels of the coagulation proteins derived from the coagulation system (20) to the system unit (14) and to receive the estimated value of the parameter of the coagulation system (20) from the system unit (14), and wherein the user interface (16) is configured to receive by a user (22) using the decision support device (10) as input to the decision support device (10) the concentration levels of the set of coagulation proteins of the coagulation system (20) and to provide to the user (22) using the decision support device (10) as output of the decision support device (10) the value of the parameter estimated by the system unit (14).

15. The method according to claim 13 or 14, wherein the set of proteins comprises 5 or 6 coagulation proteins.

16. A computer program for estimating a value of a parameter of a coagulation system (20), wherein the computer program comprises program code for causing a processor to carry out the method as defined in any of claims 1 to 12, when the computer program is run on the processor.

17. Computer readable medium (24) comprising a computer program according to claim 16.

~100

~200

# FIG. 1

100

200

300

FIG. 2

FIG. 3

FIG. 4

FIG. 5

FIG. 6

Europäisches
Patentamt

European
Patent Office

Office européen
des brevets

# EUROPEAN SEARCH REPORT

**Application Number**

EP 17 17 8329

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/60409 A1 (LINK ENTERPRISES CORP [PA]; NORA MATTEO DE [MC]; BLASIUS JACOB R [US]) 25 November 1999 (1999-11-25) * page 18 - page 20; claims * | 1,2,4-9, 12 | INV. G01N33/86 G01N33/49 G06F19/12 G06F19/00 |
| Y | WO 2014/064585 A1 (KONINKL PHILIPS NV [NL]) 1 May 2014 (2014-05-01) * claims * | 1-17 | |
| X | WO 2015/091115 A1 (KONINKL PHILIPS NV [NL]) 25 June 2015 (2015-06-25) * claims * | 1-17 | |
| Y | | 1-17 | |
| Y | WO 2009/142744 A2 (UNIV VERMONT [US]; MANN KENNETH G [US]; ZIEDINS KATHLEEN BRUMMEL [US];) 26 November 2009 (2009-11-26) * claims * | 1-17 | |

**TECHNICAL FIELDS SEARCHED (IPC)**

G01N
G06F

The present search report has been drawn up for all claims

| Place of search | Date of completion of the search | Examiner |
|---|---|---|
| Munich | 19 September 2017 | Vogt, Titus |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## EP 3 422 011 A1

### ANNEX TO THE EUROPEAN SEARCH REPORT
### ON EUROPEAN PATENT APPLICATION NO.

EP 17 17 8329

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2017

| Patent document cited in search report | | Publication date | Patent family member(s) | | Publication date |
|---|---|---|---|---|---|
| WO 9960409 | A1 | 25-11-1999 | AU | 3622699 A | 06-12-1999 |
| | | | WO | 9960409 A1 | 25-11-1999 |
| WO 2014064585 | A1 | 01-05-2014 | CN | 104756117 A | 01-07-2015 |
| | | | EP | 2912584 A1 | 02-09-2015 |
| | | | JP | 2016502650 A | 28-01-2016 |
| | | | RU | 2015119520 A | 20-12-2016 |
| | | | US | 2015278470 A1 | 01-10-2015 |
| | | | WO | 2014064585 A1 | 01-05-2014 |
| WO 2015091115 | A1 | 25-06-2015 | CN | 105829888 A | 03-08-2016 |
| | | | EP | 3084429 A1 | 26-10-2016 |
| | | | JP | 2017501409 A | 12-01-2017 |
| | | | US | 2016305965 A1 | 20-10-2016 |
| | | | WO | 2015091115 A1 | 25-06-2015 |
| WO 2009142744 | A2 | 26-11-2009 | US | 2009298103 A1 | 03-12-2009 |
| | | | WO | 2009142744 A2 | 26-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012172481 A1

**Non-patent literature cited in the description**

- **JAMES D. DOUKETIS et al.** Perioperative Management of Antithrombotic Therapy: Antithrombotic Therapy and Prevention of Thrombosis. American College of Chest Physicians Evidence-Based Clinical Practice Guidelines